# EUROPEAN PATENT APPLICATION

(11) **EP 1 184 069 A2**
(43) Date of publication of application: **06.03.2002**
(21) Application number: 01870178.9
(22) Date of filing: 17.08.2001
(51) Int. Cl.: B01D 53/74, B01D 53/77

(54) **Method of reducing odor from a stream**

(30) Priority: 18.08.2000 US 226376 P
(71) Applicant: THE PROCTER & GAMBLE COMPANY, Cincinnati, Ohio 45202 (US)
(72) Inventor: Demeyere, Hugo Jean Marie, 1785 Merchtem (BE); DePauw, Jacquy (NMN), 9300 Aalst (BE); DuVal, Dean Larry, Lebanon, Ohio 45036 (US); Trinh, Toan (NMN), Maineville, Ohio 45039 (US); Uchiyama, Hirotaka (NMN), Loveland, Ohio 45140 (US); Woo, Ricky Ah-Man, Hamilton, Ohio 45011 (US)
(74) Representative: Engisch, Gautier

(57) **Abstract**

A method of reducing odor in a process, the method comprising production of a first stream of material which contains odorous molecules and contacting a second stream of material with the first stream under conditions such that odorous molecules are transferred from the first stream to the second stream, and subsequently dispersing the second stream to the atmosphere, wherein either (a) cyclodextrin is contacted with the first stream prior to contact of the first stream with the second stream, or (b) cyclodextrin is contacted with the second stream prior to dispersing of the second stream into the atmosphere, whereby odor in the second stream is reduced.

## Description

### TECHNICAL FIELD

This invention relates to the use of cyclodextrin for the reduction of odor, especially malodor, in a stream of material, especially gaseous streams, which have picked up odor through contact with another stream of material, especially a liquid, generally an aqueous, odor-containing stream, and to processes in which cyclodextrin is used to reduce this odor. It also relates to such use for removal of non-malodorous and/or non-odorous volatile organic compounds ("VOCs").

### BACKGROUND OF THE INVENTION

Chemical plants of various kinds suffer from problems with odor emission, especially malodor emission. The odor often arises as a result of the production of odorous/malodorous molecules as by-products and/or the need to add volatile odorous ingredients, e.g., perfume, during processing. Various industrial processes result in emissions of gaseous streams bearing odor, especially malodor, molecules. This is a serious problem for the chemical plant and can lead to environmental and/or public relations issues.

Examples of systems in which this problem arises include those in which waste process water is cooled in a cooling tower before being recycled to the process. In a cooling tower a stream of air is passed through the hot process water in order to cool it and the heated air is released to the atmosphere. However, in cases where the process water contains malodor molecules these are very often picked up by the airstream and carried out into the atmosphere, causing malodor problems around the plant. Production facilities, such as paper mills, perfume and flovor plants, liquor distilleries, consumer products production plants, and the like, also emit odors that are undesirable and/or annoying to the surrounding areas.

Other areas where airstreams bearing malodor molecules can be a problem include municipal sewage treatment plants where aerobic degradation of sewage is encouraged by blowing a stream of air into the aqueous sewage stream. This results in malodors being picked up by the airstream and carried into the atmosphere surrounding the treatment plant. Similar systems in lagoons, animal waste processing and pulp and paper processing can lead to similar problems of discharge of malodorous airstreams.

Problems of this type are long-standing but have not yet been solved successfully. The primary previous attempt at a solution is in the cooling tower application and involves spraying a mist of perfume into the airstream prior to contact with the waste process water or after contact with the waste process water but before the airstream leaves the cooling tower. This has some effect but is not totally successful. It is also inevitably a masking effect.

A further attempted solution involves the use of a material known as O-scent THT which is claimed to eliminate mercaptan, hydrogen sulfide, ammonia and sulfur dioxide malodors. However, this too has not proved successful and is limited in its application range, since it is limited to treatment of malodor molecules whose malodor can be eliminated by oxidation.

The alternative to reducing malodor in these ways would be to modify the process so as to avoid production of malodorous molecules. This is not always possible and, when possible, is costly.

JP-A-08/010568 describes treatment of exhaust gases containing methyl bromide by contacting the exhaust gas with an aqueous solution of beta-cyclodextrin in order to prevent release of methyl bromide into the atmosphere. JP-A-05/031212 describes trapping of organic halides using cyclodextrin derivatives. JP-A-63/143926 describes treatment of odorous gases from treatment of industrial wastes, sewage or slaughterhouse effluent by scrubbing with an atomised aqueous solution containing cyclodextrin or maltodextrin. JP-A-01/046466 describes removal of odorous components such as organic solvent vapour from polluted air in chemical plants or laboratories by passing the air through an apparatus comprising means for wet treatment of the gas with a solution of cyclodextrin. JP-A-01/020849 describes cyclodextrin-impregnated silica microporous beads for removal of ammonia from odorous air from fish or meat processing plants. However, none of these documents addresses the particular problem with which the invention is concerned, which is the specific situation in which a gaseous stream is released to the atmosphere after contact with a liquid stream from which it has picked up malodor molecules.

Therefore it would be desirable to provide a means for reducing and if possible entirely eliminating the malodor problems which arise when air or other gas streams are contacted with waste process waters or other liquid streams before release of the gas stream into the atmosphere.

### SUMMARY OF THE INVENTION

The present invention relates to a method of reducing odor, especially malodor, in a process, especially an industrial process, the method comprising production of a first stream of material, preferably a liquid stream, which contains odorous molecules and contacting a second stream of material, preferably a gaseous stream, with the first stream under conditions such that odorous molecules are transferred from the first stream to the second stream, and subsequently dispersing the second stream into the atmosphere, wherein cyclodextrin is contacted with the first stream prior to contact of the first stream with the second stream, or cyclodextrin is contacted with the second stream prior to dispersing the second stream into the atmosphere, whereby odor in the second stream is reduced relative to odor in the second stream resulting from a method lacking steps (a) and/or (b).

The present invention further relates to a method of reducing volatile organic compounds ("VOCs") in a process by following the steps described herein.

### BRIEF DESCRIPTION OF THE DRAWING

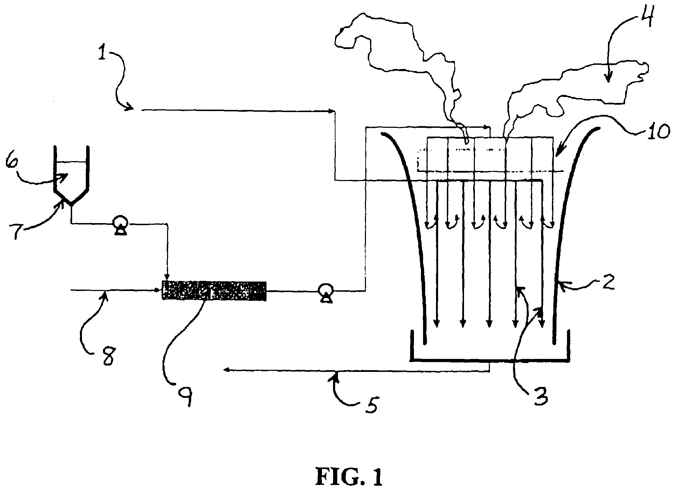

**FIG. 1** is a schematic diagram of a method of reducing odor in a process, according to the present invention.

### DETAILED DESCRIPTION OF THE INVENTION

As used herein, the term "stream" refers to materials in a phase that can be flowing, flowable, or static. Preferably, the streams described herein are flowing and are preferably liquid or gaseous. Additionally, as used herein, the terms "odorous molecules" or "odor" refer to materials that elicit a human's sense of smell. "Malodor" or "malodorous molecules" refers to odor or odorous molecules perceived by a human as unpleasant or undesirable. Also, as used herein, the term "atmosphere" refers to space (e.g. air) whereby odorous molecules and/or VOCs can be perceived by a human.

According to one embodiment of the present invention, a method of reducing odor, especially malodor, in an industrial process, the method comprising production of a liquid stream which contains odorous molecules, especially malodorous molecules, and contacting a gaseous stream with the liquid stream under conditions such that odorous molecules are transferred from the liquid stream to the gaseous stream and subsequently releasing the gaseous stream to the atmosphere, wherein cyclodextrin, preferably free, uncomplexed cyclodextrin, is contacted with the liquid stream prior to contact of the liquid stream with the gaseous stream, or contacted with the gaseous stream prior to release of the gaseous stream into the atmosphere, whereby odor in the gaseous stream is reduced.

The use of cyclodextrin in the methods of the present invention is particularly effective, especially in comparison with previously used solutions such as perfume spray and oxidising agents. Cyclodextrin is known as an complexing agent and is for instance known from US 5,668,097 as a component of compositions for domestic use in removing malodors from surfaces, but has not been suggested for use in the specific methods herein.

An example of an industrial process herein is one in which a liquid, preferably aqueous, stream containing malodor molecules is produced. The liquid stream can be a solution or a suspension. It can for instance be waste process water (which may be a suspension or dispersion but is usually a solution), cooling water, wash water, or an aqueous suspension of sewage.

In such a process, the liquid stream is brought into contact with a gaseous stream. Usually this is an airstream. The conditions of contact between the liquid stream and gaseous stream are such that odor/malodor molecules are transferred from the liquid stream to the gaseous stream. Generally this means that the contact will be under turbulent conditions in which the gaseous stream is at least partially mixed with the liquid stream. Examples of such processes include those in which an aqueous process stream is flowed along a duct under turbulent conditions, for instance subject to high shear, and an airstream passes over the aqueous process stream. The turbulent conditions result in significant mixing of the aqueous process stream with the airstream, which picks up odor/malodor and is released to the atmosphere.

A further example of an industrial process herein is a sewage treatment process. In this process an aqueous suspension of sewage is sprayed into a stream of air to encourage aerobic degradation. The airstream thus comes into contact with the aqueous sewage stream, picks up odor/malodor molecules from it and is released into the atmosphere.

Further examples of processes in which the invention may be applied include lagoon processes, animal waste processing and pulp and paper processing, where these include stages in which a gaseous stream is contacted with a liquid stream and subsequently released to the atmosphere.

Particularly advantageous applications for the methods of the current invention are processes in which the gaseous stream is passed through the liquid stream and subsequently released to the atmosphere. Examples include cooling towers in which waste process water, is cooled by passing through it a stream of air which is subsequently released to the atmosphere. The waste process water may contain malodorous molecules which are transferred to the airstream and thus to the surrounding atmosphere.

The invention can be used to reduce or eliminate odor problems with a wide variety of odor molecules, especially malodor molecules. For instance, it may be used to eliminate malodor as a result of degradation at surfactant plants, which can tend to produce fatty acid degredation and by-products thereof, which give rise to malodor problems.

In the method of the invention cyclodextrin, preferably free uncomplexed cyclodextrin, is contacted either with the liquid stream prior to its contact with the gaseous stream or with the gaseous stream itself. If contact is with the liquid stream, at least some contact with cyclodextrin should be prior to contact with the gaseous stream. However, preferably the cyclodextrin is contacted with the gaseous stream. This contact can be prior to contact of the gaseous stream with the liquid stream or subsequent to that contact, but prior to release to the atmosphere. It can occur as the gaseous stream is contacting the aqueous stream.

This invention also comprises methods to remove odor/malodor from industrial processes involving industrial and/or commercial equipment, such as farm trucks, tank cars, buses, subway trains, railway cars, fishing boats, restaurants, and the like.

The cyclodextrin, preferably uncomplexed, can be applied in any suitable form, e.g. as a solid, especially if it is to be added to a liquid or aqueous stream. However, preferably the cyclodextrin is used in the form of an aqueous solution. For instance it can be sprayed into a gaseous stream. In some applications it is preferred that the spray is in counter-current with the gaseous stream.

Generally, the sprays are applied under pressure, for instance at least about 10 bar, often at least about 20 bar.

If an aqueous cyclodextrin solution is used, the solution contacted with the liquid or gaseous stream should contain an effective amount of cyclodextrin, preferably free uncomplexed, under the usage conditions, with level of generally at least about 0.001%, preferably at least about 0.01%, by weight of the solution. Typical ranges of cyclodextrin are from about 0.001% to about 20%, preferably from about 0.005% to about 5%, more preferably from about 0.01% to 1%, and even more preferably from about 0.01% to about 0.3%, in particular from about 0.02% to about 0.1%, by weight of the solution.

In the process, the cyclodextrin solution can be delivered at the required concentration on-site. Alternatively, it may be supplied in the form of a more concentrated solution (e.g., at least about 2%, for instance at least about 5%, by weight of the solution, of cyclodextrin) which can be diluted, preferably with water, on-site prior to use.

The cyclodextrin can be supplied to a liquid stream or gaseous stream at any suitable rate, depending upon the conditions in the process as a whole. Preferably, it is supplied when in solution at a rate of from about 50 liters/hour to about 500 liters/hour, more preferably from about 100 liters/hour to about 300 liters/hour. This is particularly suitable when the cyclodextrin is sprayed into a gaseous stream, for instance in a cooling tower.

The cyclodextrin used herein is preferably in monomeric form and is preferably in water-soluble form. Solubilized, water-soluble cyclodextrin can exhibit more efficient capturing of odor molecules than non-water-soluble cyclodextrin. Monomeric cyclodextrins are those which contain only a single cavity structure per molecule. For instance, a monomeric alpha-cyclodextrin contains 6 glucose units, a monomeric beta-cyclodextrin contains 7 glucose units and a monomeric gamma-cyclodextrin contains 8 glucose units, each arranged in a donut-shaped ring.

Cyclodextrins are also known which are in polymeric form and contain more than one cyclodextrin ring per molecule, either by polymerisation of cyclodextrin or copolymerisation or grafting with other polymeric materials. These can be used in the invention but are less preferred. Further, it is also known to use cyclodextrins in water-insoluble form, usually immobilised on a carrier material. Again, cyclodextrin in this form can be used in the invention, for instance by passing the gaseous or aqueous stream across cyclodextrin immobolised on a carrier material. However, again this is less preferred.

The "cyclodextrin" may be any of the known cyclodextrins such as unsubstituted cyclodextrins containing from six to twelve glucose units, especially alpha-cyclodextrin, beta-cyclodextrin, gamma-cyclodextrin and/or their derivatives and/or mixtures thereof. The alpha-cyclodextrin consists of six glucose units, the beta-cyclodextrin consists of seven glucose units, and the gamma-cyclodextrin consists of eight glucose units, all arranged in donut-shaped rings. The specific coupling and conformation of the glucose units give the cyclodextrins a rigid, conical molecular structure with a hollow interior of a specific volume. The "lining" of each internal cavity is formed by hydrogen atoms and glycosidic bridging oxygen atoms; therefore, this surface is fairly hydrophobic. The unique shape and physical-chemical properties of the cavity enable the cyclodextrin molecules to absorb (form inclusion complexes with) organic molecules or parts of organic molecules which can fit into the cavity, especially the odorous molecules which exist in industrial process streams. Therefore, cyclodextrins, and especially mixtures of cyclodextrins with different size cavities, can be used to complex with the odor molecules. The complexation between cyclodextrin and the malodorous molecules occurs particularly rapidly in the presence of water. However, the extent of the complex formation can also depend on the polarity of the absorbed malodor molecules.

Preferably, the cyclodextrins used in the present invention are highly water-soluble, such as alpha-cyclodextrin and/or derivatives thereof, gamma-cyclodextrin and/or derivatives thereof, derivatised beta-cyclodextrins, and/or mixtures thereof. The derivatives of cyclodextrin consist mainly of molecules wherein some of the OH groups are converted to OR groups. Cyclodextrin derivatives include, for instance, those with short chain alkyl groups such as methylated cyclodextrins, and ethylated cyclodextrins, wherein R is a methyl or an ethyl group; those with hydroxyalkyl substituted groups, such as hydroxypropyl cyclodextrins and/or hydroxyethyl cyclodextrins, wherein R is a-CH₂-CH(OH)-CH₃ or a -CH₂CH₂-OH group; branched cyclodextrins such as maltose-bonded cyclodextrins; cyclodextrin glycerol ethers with structure analogous to hydroxypropyl cyclodextrins, but with the side chains forming-CH₂CH(CH₂OH)- bridges between 2' and 3' hydroxyl oxygens on the glucosyl units; cationic cyclodextrins such as those containing 2-hydroxy-3-(dimethylamino)propyl ether, wherein R is CH₂-CH(OH)-CH₂-N(CH₃)₂ which is cationic at low pH; quaternary ammonium, e.g., 2-hydroxy-3-(trimethylammonio)propyl ether chloride groups, wherein R is CH₂-CH(OH)-CH₂-N+(CH₃)₃Cl-; anionic cyclodextrins such as carboxymethyl cyclodextrins, cyclodextrin sulfates, and cyclodextrin succinylates; amphoteric cyclodextrins such as carboxymethyl/quaternary ammonium cyclodextrins; cyclodextrins wherein at least one glucopyranose unit has a 3-6-anhydro-cyclomalto structure, e.g., the mono-3-6-anhydrocyclodextrins, as disclosed in "Optimal Performances with Minimal Chemical Modification of Cyclodextrins", F. Diedaini-Pilard and B. Perly, The 7th International Cyclodextrin Symposium Abstracts, April 1994, p. 49, said references being incorporated herein by reference; and mixtures thereof. Other cyclodextrin derivatives are disclosed in U.S. Pat. Nos: 3,426,011, Parmerter et al., issued Feb. 4, 1969; 3,453,257; 3,453,258; 3,453,259; and 3,453,260, all in the names of Parmerter et al., and all issued July 1, 1969; 3,459,731, Gramera et al., issued Aug. 5, 1969; 3,553,191, Parmerter et al., issued Jan. 5, 1971; 3,565,887, Parmerter et al., issued Feb. 23, 1971; 4,535,152, Szejtli et al., issued Aug. 13, 1985; 4,616,008, Hirai et al., issued Oct. 7, 1986; 4,678,598, Ogino et al., issued Jul. 7, 1987; 4,638,058, Brandt et al., issued Jan. 20, 1987; and 4,746,734, Tsuchiyama et al., issued May 24, 1988; all of said patents being incorporated herein by reference. Further cyclodextrin derivatives suitable herein include those disclosed in V. T. D'Souza and K. B. Lipkowitz, Chemical Reviews: Cyclodextrins, Vol. 98, No. 5 (American Chemical Society, July/August 1998), which is incorporated herein by reference.

Highly water-soluble cyclodextrins are those having water solubility of at least about 10 g in 100 ml of water at room temperature, preferably at least about 20 g in 100 ml of water, more preferably at least about 25 g in 100 ml of water at room temperature.

Examples of preferred water-soluble cyclodextrin derivatives suitable for use herein are hydroxypropyl alpha-cyclodextrin, methylated alpha-cyclodextrin, methylated beta-cyclodextrin, hydroxyethyl beta-cyclodextrin, hydroxypropyl beta-cyclodextrin, hydroxypropyl gamma-cyclodextrin, and methylated gamma-cyclodextrin. Hydroxyalkyl cyclodextrin derivatives preferably have a degree of substitution of from about 1 to about 14, more preferably from about 1.5 to about 7, wherein the total number of OR groups per cyclodextrin is defined as the degree of substitution. Methylated cyclodextrin derivatives typically have a degree of substitution of from about 1 to about 18, preferably from about 3 to about 16. A known methylated beta-cyclodextrin is heptakis-2,6-di-O-methyl-beta-cyclodextrin, commonly known as DIMEB, in which each glucose unit has about 2 methyl groups with a degree of substitution of about 14. A preferred, more commercially available, methylated beta-cyclodextrin is a randomly methylated beta-cyclodextrin, commonly known as RAMEB, having different degrees of substitution, normally of about 12.6. RAMEB is more preferred than DIMEB, since DIMEB affects the surface activity of the preferred surfactants more than RAMEB. The preferred cyclodextrins are available, for instance, from Cerestar USA, Inc. and Wacker Chemicals (USA), Inc.

It can be preferred to use a mixture of cyclodextrins. Such mixtures absorb odors more broadly by complexing with a wider range of odoriferous molecules having a wider range of molecular sizes. Preferably at least a portion of the cyclodextrins is alpha-cyclodextrin and its derivatives thereof, gamma-cyclodextrin and its derivatives thereof, and/or derivatised beta-cyclodextrin; more preferably a mixture of alpha-cyclodextrin, or an alpha-cyclodextrin derivative, and derivatised beta-cyclodextrin, even more preferably a mixture of derivatised alpha-cyclodextrin and derivatised beta-cyclodextrin; and most preferably a mixture of hydroxypropyl alpha-cyclodextrin and hydroxypropyl beta-cyclodextrin, and/or a mixture of methylated alpha-cyclodextrin and methylated beta-cyclodextrin.

The cyclodextrin-containing composition used in the methods herein can contain other suitable components. A preferred optional component is perfume. When the cyclodextrin is applied in the form of an aqueous solution, suitable concentrations of perfume in the solution contacted with the liquid or gaseous stream can be at least about 0.0002% or at least about 0.0005%, and often range from about 0.0002% to about 1%, preferably from about 0.001% to about 0.3%, more preferably from about 0.002% to about 0.1%, and even more preferably from about 0.003% to about 0.02%, by weight of the solution.

If perfume is present, the typical ratio of cyclodextrin to perfume is from about 5:1 to about 20:1, preferably from about 5:1 to about 17:1, and more preferably from about 7:1 to about 10:1, by weight.

Preferably, the perfume is composed predominantly of ingredients selected from two groups of ingredients, namely, (a) hydrophilic ingredients having a ClogP of less than about 3.2, more preferably equal to or less than about 3.0, and (b) ingredients having significant low detection threshold, and mixtures thereof, wherein ClogP of a perfume is the calculated value of the logarithm to the base 10 of its octanol/water partition coefficient P. Thus, the perfume preferably contains at least about 20%, preferably at least about 30%, more preferably at least about 40%, even more preferably at least about 60%, and yet more preferably at least about 80%, by weight of the perfume of perfume ingredients that have a ClogP equal or less than about 3.2, more preferably equal to or less than about 3.0. Nonlimiting examples and preferred hydrophilic perfume ingredients and low odor detection threshold perfume ingredients, as well as methods to determine ClogP, are given in U.S. Pat. No. 5,997,759, which is incorporated herein by reference.

Other suitable optional ingredients include other adjunct odor control agents that are cyclodextrin-compatible, preferably selected from the group consisting of: soluble metallic salts, preferably zinc and copper salts; low molecular weight polyols; antimicrobial actives; and mixtures thereof. Nonlimiting examples of these cyclodextrin-compatible adjunct odor control agents are also given in U.S. Pat. No. 5,997,759, which is incorporated herein by reference. When these adjunct odor control agents are added to the cyclodextrin solutions herein, they are typically present at a level of from about 0.001% to about 0.5%, preferably from about 0.005% to about 0.2%, and more preferably from about 0.01% to about 0.1%, by weight of the solution. Other useful optional cyclodextrin-compatible ingredients include surfactants, algaecides, antimicrobial preservatives, chelating agents, enzymes, enzyme inhibitors, and mixtures thereof, as are described in U.S. Pat. No. 5,997,759, which is incorporated herein by reference. In this context "cyclodextrin-compatible" means that the cyclodextrin and the other material, or active, do not substantially interact so as to eliminate the odor controlling ability of the cyclodextrin or the desired effect of the other material or active.

Cyclodextrin can also be useful in eliminating volatile organic compounds (VOCs) from streams of material produced in the above manner, even when those VOCs are not odorous molecules. Thus another aspect of the current invention includes a method of reducing VOCs emitted by a process, preferably an industrial process, the method comprising production of a first stream of material, preferably a liquid stream, which contains VOC molecules and contacting a second stream of material, preferably a gaseous stream, with the first stream under conditions such that VOC molecules are transferred from the first stream to the second stream, and subsequently dispersing the second stream into the atmosphere, wherein cyclodextrin is contacted with the first stream prior to contact of the first stream with the second stream or contacted with the second stream prior to dispersing of the second stream into the atmosphere, whereby VOC content in the second stream is reduced.

Thus, this aspect of the invention solves problems which can be caused by release of VOCs into the atmosphere which may not be malodorous but nevertheless harmful to the environment. All aspects of the invention discussed above may be applied where appropriate.

The invention will now be illustrated with reference to the following examples.

### EXAMPLES

**Figure 1** shows a schematic diagram of a system in which the invention is used. In the system shown, waste process water **1** passes into cooling tower **2.** As it passes down the cooling tower, streams of air **3** (generated by the rotating blades **10)** pass up the tower **2** through the waste process water **1,** cooling it, and are subsequently released to the atmosphere (at **4**). The cooled water is then recirculated (at **5**) to the process. As the airstreams **3** pass through the process water **1**, they pick up odor molecules. For instance, at a surfactant plant, the malodor may be caused by by-products which can result from fatty acid degradation. As a result, an odor problem, especially a malodor problem, arises around the plant and in neighbouring communities.

In order to solve this problem a cyclodextrin solution **6** is made up in a make-up tank **7**. This is fed at a rate of about 1 liter/hour to a water stream **8**. The water and cyclodextrin solution **6** are mixed at a mixing stage **9** to form a stream of dilute cyclodextrin solution which passes into the cooling tower air stream **3** at a rate of about 200 liters/hour. The dilute cyclodextrin solution is sprayed into the airstreams **3** as it contacts the waste process water **1**. In this embodiment, it is applied in counter-current with the airstreams **3**. The spray is applied at a pressure of about 30 bar. Use of the present methods in this system can substantially eliminate malodor problems emanating from the cooling tower.

In an alternative embodiment the spray of dilute cyclodextrin solution is applied at the bottom of the tower **2**, in co-current with the airstreams **3**.

Example solutions injected into the cooling tower **2** have a level of cyclodextrin of (a) about 0.01% and (b) about 0.02%. These two examples substantially eliminate malodor problems.

A further example solution (c) contains about 0.06% cyclodextrin plus about 0.01% perfume. This completely eliminates malodor. A further example (d) of about 0.012% cyclodextrin plus about 0.002% perfume reduces but does not completely eliminate malodor. A further test using (e) about 0.02% cyclodextrin and about 0.004% perfume also completely eliminates malodor.

## Claims

1. A method of reducing odor in a process, the method comprising production of a first stream of material which contains odorous molecules and contacting a second stream of material with the first stream under conditions such that odorous molecules are transferred from the first stream to the second stream, and subsequently dispersing the second stream to the atmosphere, wherein either (a) cyclodextrin is contacted with the first stream prior to contact of the first stream with the second stream, or (b) cyclodextrin is contacted with the second stream prior to dispersing of the second stream into the atmosphere, whereby odor in the second stream is reduced relative to odor in the second stream resulting from a method lacking steps (a) and/or (b).

2. A method of reducing volatile organic compound emissions from a process, the method comprising production of a first stream of material which contains volatile organic compounds and contacting a second stream of material with the first stream under conditions such that volatile organic compounds are transferred from the first stream to the second stream, and subsequently dispersing the second stream to the atmosphere, wherein either (a) cyclodextrin is contacted with the first stream prior to contact of the first stream with the second stream, or (b) cyclodextrin is contacted with the second stream prior to dispersing of the second stream into the atmosphere, whereby the level of volatile organic compounds in the second stream is reduced relative to the level of volatile organic compounds in the second stream resulting from a method lacking steps (a) and/or (b).

3. The method according to claim 1 or claim 2 wherein each of the first stream and the second stream is either a liquid stream or a gaseous stream.

4. The method according to any one of the preceding claims wherein the first stream is a liquid stream and the second stream is a gaseous stream.

5. The method according to claim 4 wherein the liquid stream is an aqueous solution, aqueous dispersion or aqueous suspension.

6. The method according to claim 4 wherein the liquid stream is waste process water.

7. The method according to any one of claims 4-6 wherein the liquid stream is brought into contact with the gaseous stream under turbulent conditions in which the gaseous stream is at least partially mixed with the liquid stream.

8. The method according to any one of claims 4-7 wherein the gaseous stream is passed through the liquid stream and subsequently dispersed into the atmosphere.

9. The method according to any one of claims 4-8 wherein the cyclodextrin is contacted with the gaseous stream.

10. The method according to claim 9 wherein the cyclodextrin is contacted with the gaseous stream prior to contact of the gaseous stream with the liquid stream.

11. The method according to claim 9 wherein the cyclodextrin is contacted with the gaseous stream after contact of the gaseous stream with the liquid stream.

12. The method according to any one of the preceding claims wherein the process is an industrial process in which the first stream is waste process water that is cooled by the second stream, wherein the second stream is airstreams in a cooling tower.

13. The method according to any one of the preceding claims wherein the cyclodextrin is selected from the group consisting of beta-cyclodextrin, alpha-cyclodextrin, gamma-cyclodextrin, derivatives of said cyclodextrins, and mixtures thereof; wherein said cyclodextrin derivatives are preferably selected from the group consisting of methyl substituted cyclodextrins, ethyl substituted cyclodextrins, hydroxyalkyl substituted cyclodextrins, cyclodextrin glycerol ethers; branched cyclodextrins, cationic cyclodextrins, quatemary ammonium cyclodextrins, anionic cyclodextrins, amphoteric cyclodextrins, cyclodextrins wherein at least one glucopyranose unit has a 3-6-anhydro-cyclomalto structure, and mixtures thereof, more preferably hydroxypropyl beta-cyclodextrin, methylated beta-cyclodextrin, hydroxypropyl alpha-cyclodextrin, methylated alpha-cyclodextrin, and mixtures thereof.

14. The method according to any one of the preceding claims wherein the cyclodextrin is used in the form of an aqueous solution.

15. The method according to claim 14 wherein the aqueous solution of cyclodextrin is contacted with the first stream or second stream in the form of a spray.

16. The method according to claim 14 or claim 15 wherein the concentration of the cyclodextrin in the aqueous solution is from about 0.001% to about 20%, preferably from about 0.005% to about 5%, more preferably from about 0.01% to 1%, even more preferably from about 0.01% to about 0.3%, in particular from about 0.02% to about 0.1%, by weight of the solution.

17. The method according to any one of the preceding claims wherein the cyclodextrin is in monomeric form.

18. The method according to any one of the preceding claims wherein the cyclodextrin is used in the form of an aqueous solution which additionally contains perfume in an amount of from about 0.0002% to about 1%, preferably from about 0.001% to about 0.3%, more preferably from about 0.002% to about 0.1%, and even more preferably from about 0.003% to about 0.02%, by weight of the solution.

19. The method according to any one of the preceding claims wherein the cyclodextrin is used in the form of an aqueous solution which additionally comprises a cyclodextrin-compatible adjunct odor control agent, preferably selected from the group consisting of: soluble metallic salts, preferably zinc and copper salts; low molecular weight polyols; antibacterial actives; and mixtures thereof; and present at a level from about 0.001% to about 0.5%, preferably from about 0.005% to about 0.2%, and more preferably from about 0.01% to about 0.1%, by weight of the solution.

20. The method according to any one of the preceding claims wherein the cyclodextrin is used in the form of an aqueous solution which additionally comprises a cyclodextrin-compatible ingredient selected from the group consisting of surfactants, algaecides, antimicrobial preservatives, chelating agents, enzymes, enzyme inhibitors, and mixtures thereof.

21. Use of cyclodextrin in a process containing at least two separate streams of material to reduce odor in at least one of the streams in the process.
